# EUROPEAN PATENT SPECIFICATION

(11) **EP 4 515 212 B1**
(45) Date of publication and mention of the grant of the patent: **07.10.2026**
(21) Application number: 23724675.6
(22) Date of filing: 28.04.2023
(51) Int. Cl.: G01N 21/64, G02B 21/08

(54) **ILLUMINATION UNIT FOR FLUORESCENCE IMAGING APPARATUS**
BELEUCHTUNGSEINHEIT FÜR FLUORESZENZBILDGEBUNGSVORRICHTUNG
UNITÉ D'ÉCLAIRAGE POUR APPAREIL D'IMAGERIE PAR FLUORESCENCE

(30) Priority: 29.04.2022 EP 22170826
(43) Date of publication of application: 05.03.2025
(73) Proprietor: SurgVision GmbH, 81379 Munich (DE)
(72) Inventor: SHARAPOV, Anton, 81379 Munich (DE); KOCH, Maximilian, 81379 Munich (DE); TARUTTIS, Adrian, 81379 Munich (DE); MANDON, Julien Benoît, 7521 PH Enschede (NL); SIMMELINK, Kevin, 7521 PH Enschede (NL)
(74) Representative: Pezzoli, Ennio
(86) International application number: PCT/EP2023/061326
(87) International publication number: WO 2023/209195

(56) References cited:
- EP-A1- 3 858 215
- WO-A1-2020/014786
- WO-A1-2022/023332
- CN-A- 106 092 994
- US-A1- 2007 024 946
- US-A1- 2010 193 705
- US-A1- 2012 049 089

## Description

### Technical field

The present disclosure relates to the field of medical equipment. More specifically, this disclosure relates to fluorescence imaging apparatus.

### Background art

The background of the present disclosure is hereinafter introduced with the discussion of techniques relating to its context. However, even when this discussion refers to documents, acts, artifacts and the like, it does not suggest or represent that the discussed techniques are part of the prior art or are common general knowledge in the field relevant to the present disclosure.

Imaging apparatus are commonly used in several medical applications to provide visual representations of body-parts of patients even if they are not visible directly. Particularly, imaging apparatus of fluorescence type exploit a fluorescence phenomenon occurring in fluorescence substances (called fluorophores), which emit (fluorescence) light when they are illuminated. Images of the body-parts defined by the fluorescence light that is emitted from different locations of the body-parts (fluorescence images) then represent the fluorophores that are present therein. For example, fluorescence agents (possibly adapted to reaching specific molecules of desired targets, such as lesions like tumors, and then to remaining immobilized thereon in Fluorescence Molecular Imaging (FMI) applications) may be administered to the patients. The representation of the (immobilized) fluorescence agents in the corresponding fluorescence images then facilitates the identification (and quantification) of the corresponding targets. This information may be used in several medical applications, for example, in surgical applications for recognizing margins of lesions to be resected, in diagnostic applications for discovering/monitoring lesions and in therapeutic applications for delineating lesions to be treated.

For this purpose, the imaging apparatus is provided with an illumination unit that provides an excitation light required to excite the fluorophores of interest *(i.e.,* of the fluorescence agents in FMI applications). In order to obtain accurate fluorescence images, it is important to illuminate a field of view of the imaging apparatus as homogeneously as possible (since any changes in its illumination lead to spurious effects in the fluorescence images).

A laser may be used in the illumination unit to obtain a high illumination homogeneity of the field of view. However, the laser is relatively expensive and bulky, and it requires a relatively complex control circuit; moreover, the laser is difficult to implement for a wide field of view and it generates speckles.

Alternatively, Light Emitting Diodes (LEDs) may be used in place of the laser. The LEDs are less expensive, bulky and complex.

However, the LEDs have a relatively low power, so that multiple LEDs are required to obtain an intensity of the illumination necessary to excite the fluorophores. Moreover, the Etendue (area of a light source multiplied by a solid angle of its illumination pattern) of the LEDs is generally far larger than that of a laser.

Generally, it is difficult to obtain a satisfactory illumination homogeneity of the field of view with the LEDs. This is further exacerbated by the fact that typically it is not possible to distribute the LEDs uniformly in the illumination unit because of the need of arranging collection optics (of an acquisition unit of the imaging apparatus) at a center thereof.

At the same time, it is difficult to concentrate the excitation light of the LEDs entirely towards the field of view. Therefore, a larger area is flooded with the excitation light; this involves a waste of power and it may cause additional spurious effects in the fluorescence images.

Moreover, the multiple LEDs hinder the addition of further light sources to the illumination unit. Particularly, it is difficult to provide white light sources used to illuminate any objects present in the field of view for acquiring corresponding reflectance images.

Likewise, the multiple LEDs also hinder the addition of (optical) excitation filters required to limit a frequency band of the excitation light. Particularly, it is difficult to provide excitation filters of small size covering all the LEDs, with a corresponding increase of material and then of costs.

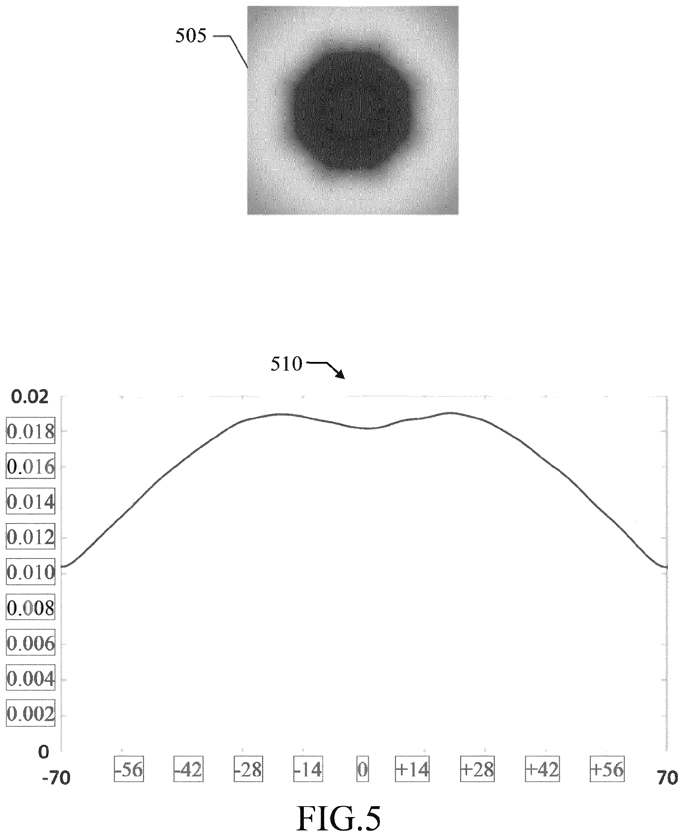

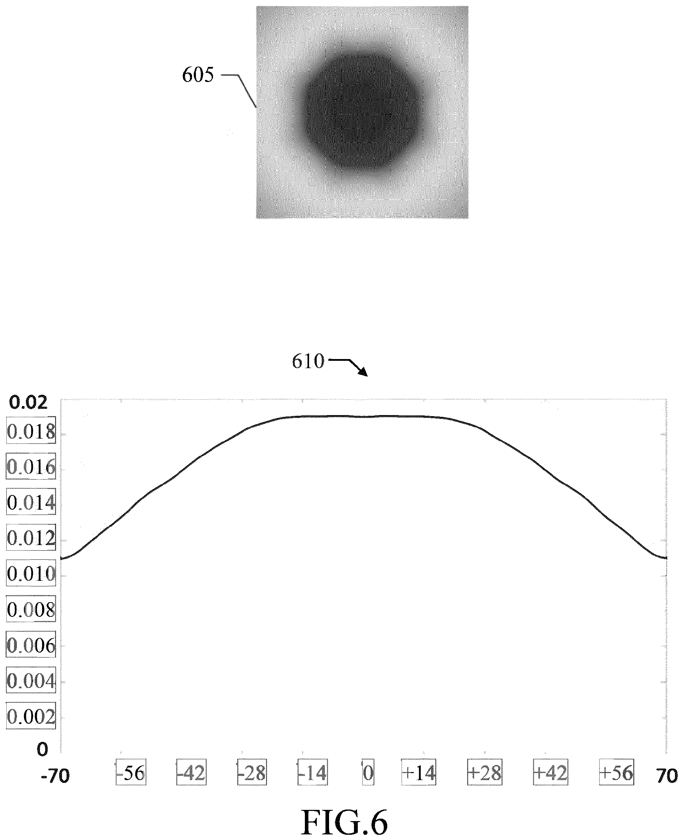

US-A-2010/193705 A1 describes an apparatus for biochemical analysis of samples. Particularly, two mutually exclusive implementations are shown. In a first case (Fig.5), two equal excitation light sources being inclined towards the sample and a completely separated detector system are arranged on a same side of the sample; in a second case (Fig.6A), instead, an alignment of excitation light sources with different diffusion angles and an imaging sensor are arranged at opposite sides of the sample, both of them at a center thereof.

WO-A-2020/014786 A1 describes a fluorescence imaging apparatus with radial alignments of LEDs providing lights having different colors. US-A-2007/024946 A1 describes a hyperspectral/multispectral imaging system with radial alignments of LEDs providing lights of different wavelengths. US-A-2012/049089 A1 describes an assembly (having a chamber for receiving objects/specimens to be imaged), which is provided with light sources of different wavelengths that are turned on selectively.

### Summary

A simplified summary of the present disclosure is herein presented in order to provide a basic understanding thereof; however, the sole purpose of this summary is to introduce some concepts of the disclosure in a simplified form as a prelude to its following more detailed description, and it is not to be interpreted as an identification of its key elements nor as a delineation of its scope.

In general terms, the present disclosure is based on the idea of using excitation sources with different emission characteristics.

Particularly, an aspect provides an illumination unit as defined in claim 1 for use in a fluorescence imaging apparatus. The illumination unit comprises excitation sources arranged in alignments, which extend radially from a hole of the illumination unit for receiving collection optics of an acquisition unit. The excitation sources of each alignment are configured to have at least in part different emission characteristics of their excitation light.

A further aspect provides an imaging head comprising the illumination unit.

A further aspect provides a fluorescence imaging apparatus comprising the imaging head.

A further aspect provides a method for imaging a body-part of a patient with the fluorescence imaging apparatus.

More specifically, one or more aspects of the present disclosure are set out in the independent claims and advantageous features thereof are set out in the dependent claims, with the wording of all the claims that is herein incorporated *verbatim* by reference (with any advantageous feature provided with reference to any specific aspect that applies *mutatis mutandis* to every other aspect).

### Brief description of the drawings

The solution of the present disclosure, as well as further features and the advantages thereof, will be best understood with reference to the following detailed description thereof, given purely by way of a non-restrictive indication, to be read in conjunction with the accompanying drawings (wherein, for the sake of simplicity, corresponding elements are denoted with equal or similar references and their explanation is not repeated, and the name of each entity is generally used to denote both its type and its attributes, such as value, content and representation). In this respect, it is expressly intended that the drawings are not necessary drawn to scale (with some details that may be exaggerated and/or simplified) and that, unless otherwise indicated, they are merely used to illustrate the structures and procedures described herein conceptually. In addition, orientations and related position references (such as front, rear, upper, lower, lateral and so on) are to be understood in relation to a condition of use of the corresponding entities. Particularly:
FIG. 1 shows a pictorial representation of a fluorescence imaging apparatus wherein the solution according to an embodiment of the present disclosure may be used,
FIG.2 shows a functional block diagram of an imaging head wherein the solution according to an embodiment of the present disclosure may be implemented,
FIG.3 shows a schematic representation in bottom view and in cross-section view of an illumination unit according to an embodiment of the present disclosure,
FIG.4 shows a functional representation of an excitation source alignment according to an embodiment of the present disclosure, and
FIG.5-FIG.7 show different examples of experimental results relating to the solution according to an embodiment of the present disclosure.

### Detailed description

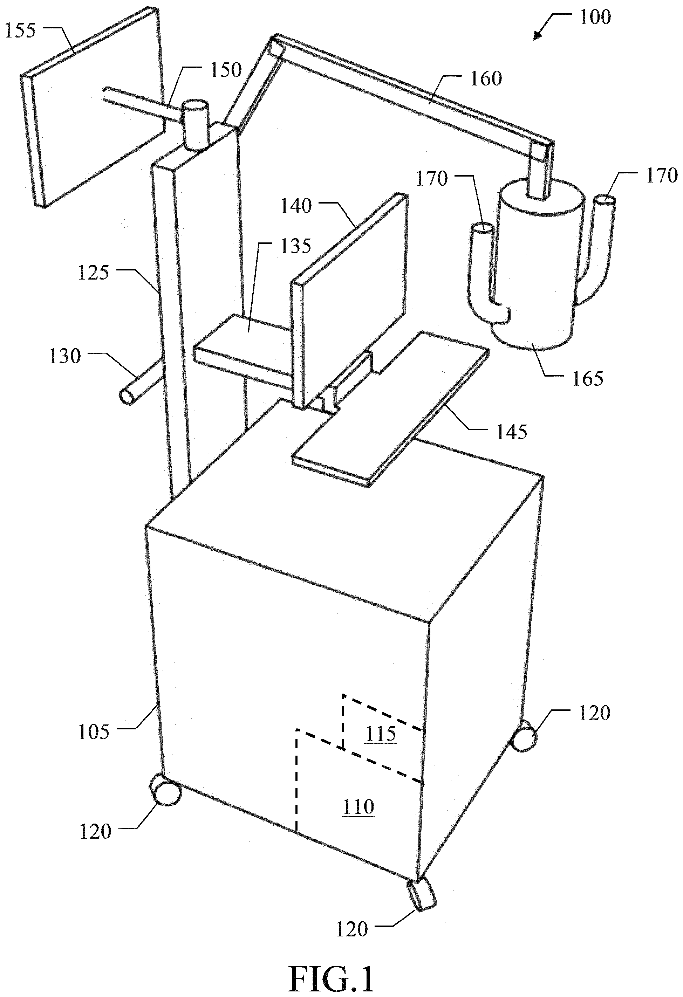

With reference in particular to FIG.1, a pictorial representation is shown of a fluorescence imaging apparatus 100 wherein the solution according to an embodiment of the present disclosure may be used.

The (fluorescence) imaging apparatus 100 is used in medical applications to inspect body-parts of patients (not shown in the figure), for example, for diagnostic, therapeutic and/or surgical purposes, by applying fluorescence imaging techniques. For example, the imaging apparatus 100 is used to assist a surgeon in Fluorescence Guided Surgery (FGS), and particularly Fluorescence Guided Resection (FGR) when relating to tumors.

The imaging apparatus 100 comprises the following components. A trolley 105 houses a supply unit 110 and a control unit 115 for supplying and controlling, respectively, the imaging apparatus 100. For example, not shown in the figure, the control unit 115 is based on a microprocessor (providing the logic capability of the control unit 115), which microprocessor is associated with a non-volatile memory storing a control program, a volatile memory used as working memory and drives for corresponding peripherals of the imaging apparatus 110. Four casters 120 (only three visible in the figure) are arranged at corresponding lower corners of the trolley 105 to facilitate moving the imaging apparatus 100 (with a foot brake, not shown in the figure, that is provided for securing the imaging apparatus 100 in position). A pillar 125 extends upwards from a back surface of the trolley 105. The pillar 125 has a handlebar 130 for moving the imaging apparatus 100 by an operator thereof. A cantilever 135 projects from the pillar 125, above the trolley 105. A primary monitor 140 (for displaying images to the operator) and a keyboard 145 with a pointing device such as a mouse or a trackball (for entering information/commands by the operator) are mounted on the cantilever 135. A pivoting arm 150 is mounted on top of the pillar 125 (above the cantilever 135). A secondary monitor 155 (for displaying images to a doctor, such as a surgeon) is mounted on the pivoting arm 150 (so as to allow turning it in any directions). An articulated arm 160 is mounted on top of the pillar 125 as well (next to the pivoting arm 150). An imaging head 165 (for imaging the body-parts under analysis) is suspended from the articulated arm 160. The imaging head 165 is provided with two handlebars 170 for positioning it by the operator.

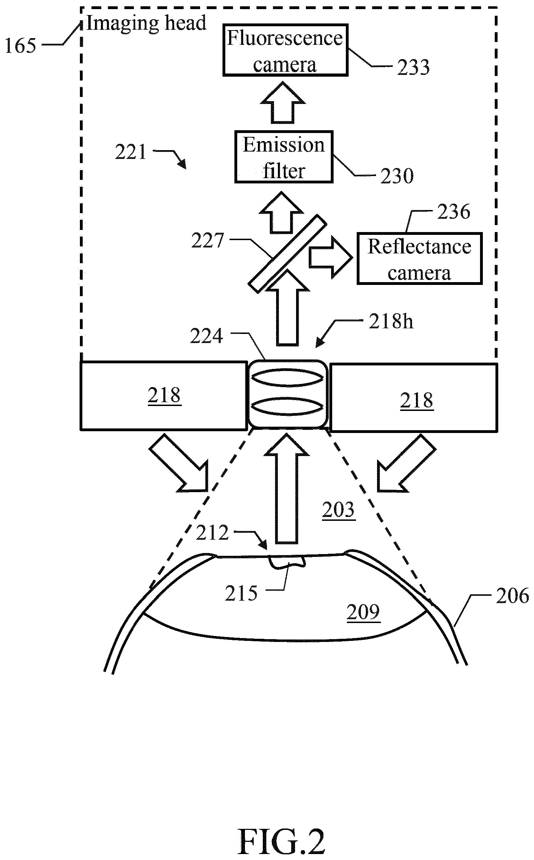

With reference now to FIG.2, a functional block diagram is shown of the imaging head 165 wherein the solution according to an embodiment of the present disclosure may be implemented.

The imaging head 165 is configured for imaging a scene comprised in a field of view 203 thereof (defined by a part of the world within a solid angle to which the imaging head 165 is sensitive). Particularly, in case of surgical applications the scene relates to a patient 206 undergoing a surgical procedure, to whom a fluorescence agent has been previously administered (for example, adapted to accumulating in tumors). The scene comprises a body-part 209 of the patient 206, wherein a surgical cavity 212 (for example, a small skin incision in minimally invasive surgery) has been opened to expose a tumor 215 to be resected.

The imaging head 165 comprises the following components. An illumination unit 218 (described in detail in the following) is used to illuminate the scene of the field of view 203. Particularly, the illumination unit 218 generates an excitation light and a white light; the excitation light has wavelength and energy suitable to excite the fluorophores of the fluorescence agent (such as of Near Infra-Red (NIR) type), whereas the white light appears substantially colorless to the human eye (such as containing all the wavelengths of the spectrum that is visible to the human eye at equal intensity). An acquisition unit 221 is used to acquire (digital) images of the scene of the field of view 203. The acquisition unit 221 comprises the following components. Collection optics 224 is received in a hole 218h of the illumination unit 218 for collecting light from the field of view 203 (in an epi-illumination geometry). The collected light comprises fluorescence light that is emitted by any fluorophores present in the field of view 203 (illuminated by the excitation light). Indeed, the fluorophores pass to an excited (electronic) state when they absorb the excitation light; the excited state is unstable, so that the fluorophores very shortly decay therefrom to a ground (electronic) state, thereby emitting the fluorescence light (at a characteristic wavelength, longer than the one of the excitation light because of energy dissipated as heat in the excited state) with an intensity mainly depending on the amount of the fluorophores that are illuminated. Moreover, the collected light comprises visible light (being visible to the human eye) that is reflected (including diffused) by any object present in the field of view 203 (illuminated by the white light). A beam-splitter 227 splits the collected light into two channels. For example, the beam-splitter 227 is a dichroic mirror transmitting and reflecting the collected light at wavelengths above and below, respectively, a threshold wavelength between a spectrum of the visible light and a spectrum of the fluorescence light (or *vice-versa).* In the (transmitted) channel of the beam-splitter 227 with the fluorescence light defined by the portion of the collected light in its spectrum, an emission filter 230 filters the fluorescence light to remove any residual component thereof outside the spectrum of the fluorescence light. A fluorescence camera 233 (for example, of EMCCD or CMOS type) receives the fluorescence light from the emission filter 230 and generates a corresponding fluorescence (digital) image representing the distribution of the fluorophores in the field of view 203. In the other (reflected) channel of the beam-splitter 227 with the visible light defined by the portion of the collected light in its spectrum, a reflectance, or photograph, camera 236 (for example, of CCD or CMOS type) receives the visible light and generates a corresponding reflectance (digital) image representing what is visible to the human eye in the field of view 203.

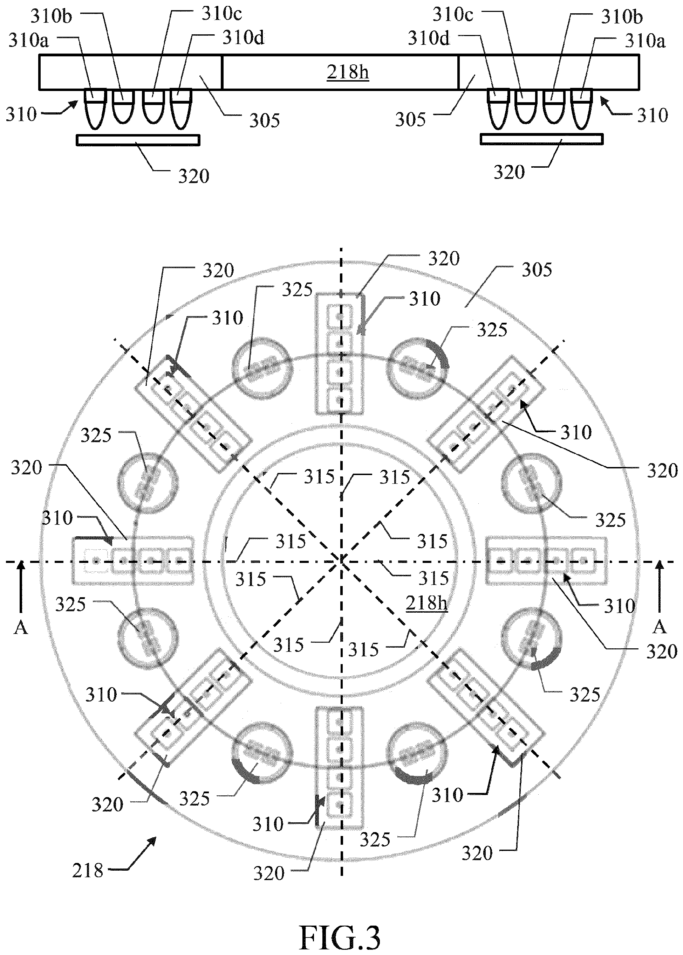

With reference now to FIG.3, a schematic representation is shown in bottom view and in cross-section view of the illumination unit 218 according to an embodiment of the present disclosure.

Particularly, the bottom view represents the illumination unit 218 as seen from an operative side of the imaging head (without any sterile cover that may enclose the whole imaging head), which operative side is generally facing downwards during an imaging procedure. The cross-section view represents the illumination unit 218 as taken along the cutting plane A-A.

The illumination unit 218 comprises the following components. A base 305 (a disk in the example shown in the figure) supports (mechanically) the other components of the illumination unit 218. The base 305 has a though-hole (for example, with a circular shape at a center thereof) that defines the hole 218h used to receive the collection optics of the acquisition unit (not shown in the figure). A plurality of excitation light sources, or simply excitation sources 310 provide the excitation light, which has a common wavelength (for example, 800-2,500 nm) for all the excitation sources 310. In the example shown in the figure, there are 32 excitation sources 310 (such as each formed by an excitation LED with a corresponding lens). The excitation sources 310 are arranged in a plurality of alignments 315. The (excitation source) alignments 315 extends radially (in a star-like configuration) from the hole 218h in a uniform way, *i.e.,* spaced apart angularly by a constant value. In the example shown in the figure, the excitation source alignments 315 are distributed (uniformly) along an annulus in a peripheral area of the base 305. Each excitation source alignment 315 comprises a plurality of excitation sources 310. In the example shown in the figure, each excitation source alignment 315 comprises 4 excitation sources 310, differentiated with the references 310a, 310b, 310c and 310d (so that the 32 excitation sources 310 are distributed in 8 excitation source alignments 315 spaced apart radially by 45°).

In the solution according to an embodiment of the present disclosure, as described in detail in the following, in each excitation source alignment 315, its excitation sources 310 have at least in part different emission characteristics of the excitation light. For example, the excitation sources 310b,310c have a higher radiation angle than the excitation sources 310a,310d.

The above-mentioned solution allows maintaining an optimal arrangement of the collection optics (particularly, at the center of the illumination unit), at the same time obtaining a relatively high illumination homogeneity of the field of view (even in case of multiple LEDs having relatively low power and despite their non-uniform distribution). This has a beneficial effect on a quality of the fluorescence images that are acquired (since it reduces spurious effects therein).

At the same time, the excitation light is concentrated mainly towards the field of view. This reduces a waste of power and additional spurious effects in the fluorescence images.

Specific implementations of the illumination unit 218 according to an embodiment of the present disclosure provide additional advantageous effects.

Particularly, the excitation source alignments 315 are equal to each other, *i.e.,* same number of same excitation sources 310 being arranged in the same way *(i.e.,* at a same distance from the hole 218h, with the corresponding excitation sources 310a, 310b, 310c and 310d of the different excitation source alignments 315 being positioned along corresponding concentric circumferences that are equal to each other).

This further improves the illumination homogeneity of the field of view.

Corresponding (optical) excitation filters 320 cover the excitation source alignments 315 (below them in the figure); the excitation filters 320 limit a frequency band of the excitation light that is emitted by the corresponding excitation sources 310 (for example, in a range from 700-900 nm to 2.400-2.600 nm). Each excitation filter 320 is configured as a strip, with a generic rectangular shape; the excitation filter 320 has a size slightly larger than a footprint of the corresponding excitation sources 310 (4 in the example at issue).

As a result, the excitation filters 320 may be maintained relatively small. This allows reducing an amount of material required by the excitation filters 320, and then their cost.

A plurality of white light sources, or simply white sources 325 provide the white light. The white sources 325 are interposed among the excitation source alignments 315 in a uniform way, *i.e.,* in the middle of each pair of adjacent excitation source alignments 315. In the example shown in the figure, there are 16 white sources 325 (such as each formed by a white LED with a power of 1-3 W being covered by a corresponding lens); the white sources 325 are arranged along a circumference at a center of the annulus of the excitation source alignments 315, in groups each of 2 white sources 325 close to each other.

This result is achieved by exploiting the room that is already available among the excitation source alignments 315. Therefore, the addition of the white sources 325 is possible without any increase in size of the illumination unit 218.

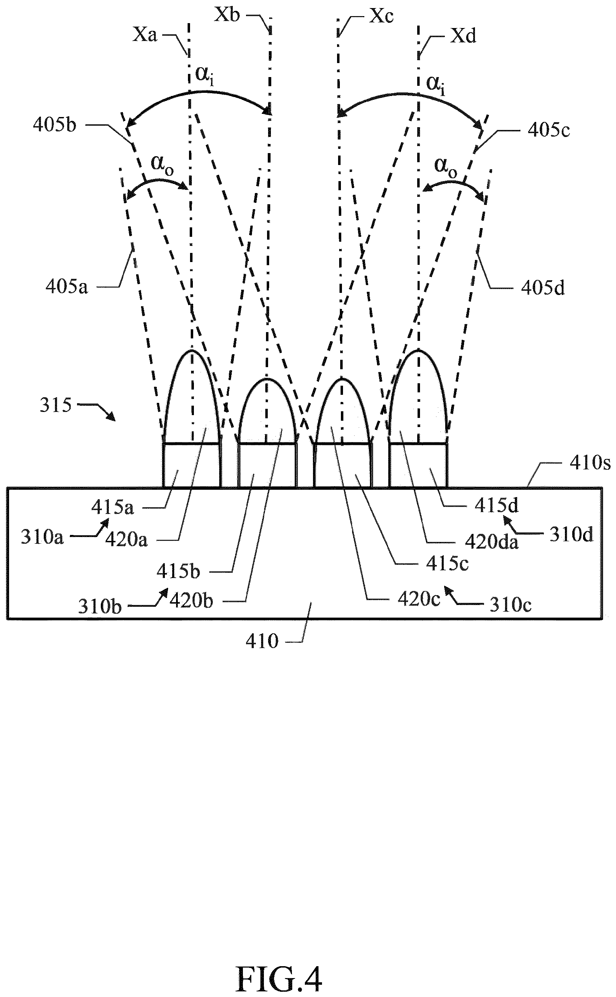

With reference now to FIG.4, a functional representation is shown of an excitation source alignment 315 according to an embodiment of the present disclosure.

In a specific implementation, the excitation sources 310a-310d (turned upside down with respect to FIG.3) emit the excitation light with at least in part different spatial distributions, and particularly different radiation angles. The radiation angle of each excitation source 310a, 310b, 310c and 310d is defined by a corresponding emission cone 405a, 405b, 405c and 405d, respectively, of the excitation light that is emitted by it. A boundary of the emission cone 405a-405d is determined by a position at which an irradiance of the excitation source 310a-310d (radiant flux being received per unit area) decreases by a pre-defined amount (such as -3dB, *i.e.,* about 50%) from its maximum value. The radiation angle is measured by half a vertex angle (half-angle) of the emission cone 405a-450d.

Particularly, the excitation sources 310a-310d are mounted on a Printed Circuit Board (PCB), or simply board 410, which physically supports and electrically connects them (for example, a single board 410 with an annulus-like shape for all the excitation source alignments 315). The emission cones 405a, 405b, 405c and 405d are symmetric, with their axes of symmetry Xa, Xb, Xc and Xd, respectively, that extend perpendicularly to a (common) surface 410s of the board 410 wherein the excitation sources 310a-310d are mounted. This significantly simplifies the mounting of the excitation sources 310a-310d on the board 410.

The (outer) excitation sources 310a,310d (extending from the ends of the excitation light alignment 315, *i.e.,* located at opposite edges of the excitation light alignment 315) have their emission cones 405a,405d with a same (outer) half-angle αₒ; the (inner) excitation sources 310b,310c (being surrounded by the outer excitation sources 310a,3105d along the excitation light alignment 315, *i.e.,* interposed between them) have their emission cones 405b,405c with a same (inner) half-angle αᵢ (different from the outer half-angle αₒ;); the inner half-angle αᵢ is (strictly) higher than the outer half-angle αₒ (*i.e.,* αᵢ>αₒ). This provides a symmetric arrangement of the excitation sources 310 along the excitation source alignment 315, thereby reducing any mechanical stress on the board 410 (due to thermal deformations caused by the heat dissipated by the excitation sources 310).

Particularly, the inner half-angle αᵢ is preferably 1.9-3.1 times, more preferably 2.2-2.8 times and still more preferably 2.4-2.6 times, such as 2.5 times, the outer half-angle αₒ. In absolute terms, the inner half-angle αᵢ is preferably 16°-28°, more preferably 19°-25° and still more preferably 21°-23°, such as 22°, whereas the outer half-angle αₒ is preferably 3°-15°, more preferably 6°-12° and still more preferably 8°-10°, such as 9°. These values have been found to provide optimal results in terms of homogeneity and concentration of the illumination of the field of view.

For example, the excitation sources 310a, 310b, 310c and 310d comprise corresponding (excitation) LEDs 415a, 415b, 415c and 415d, respectively (mounted on the board 410 with surface mounting technology). The LEDs 415a-415d are equal to each other (for example, with a power of 0.5-3.0 W, such as 0.8 W, in continuous mode or a power of 1.0-4.0 W, such as 1.7 W in pulsed mode). The LEDs 415a-415d approximate corresponding Lambertian radiators, which emit the excitation light with a uniform radiance (radiant flux per unit solid angle per unit area) in all directions. The LEDs 415a, 415b, 415c and 415d are covered by corresponding lenses 420a, 420b, 420c and 420d, respectively (for example, made of silicone resin). The lenses 420a,420d of the outer excitation sources 310a,310d are equal to each other, and the lenses 420b,420c of the inner excitation sources 310b,310c are equal to each other (and different from the lenses 420a,420d); the lenses 420a,420d are shaped to reduce the emission cones 405a,405d of the corresponding LEDs 415a,415d to the outer half-angle αₒ, and the lenses 420b,420c are shaped to reduce the emission cones 405b,405c of the corresponding LEDs 415b,415c to the inner half-angle αᵢ. This implementation is particular simple and cost-effective.

In addition or in alternative, the excitation sources 310a-310d emit the excitation light with at least in part different radiant intensities (radiant flux per unit solid angle). Particularly, the outer excitation sources 310a,310d emit the excitation light with a same (outer) radiant intensity *Io*, whereas the inner excitation sources 310b,310c emit the excitation light with a same (inner) radiant intensity *Ii* (different from the outer radiant intensity *Io*); the outer radiant intensity *Io* (of the outer excitation sources 310a,310d with lower outer half-angle αₒ) is (strictly) higher than the inner radiant intensity *Ii* (*i.e., Io>Ii*). Particularly, the outer radiant intensity *Io* is preferably 1.4-2.3 times, more preferably 1.5-2.1 times and still more preferably 1.63-1.94 times, such as 1.78 times, the outer radiant intensity *Io*. In absolute terms, the outer radiant intensity *Io* is preferably 2,900-3,500 W/sr, more preferably 3,000-3,400 W/sr and still more preferably 3,100-3,300 W/sr, such as 3,200 W/sr, whereas the inner radiant intensity *Ii* is preferably 1,500-2,100 W/sr, more preferably 1,600-2,00 W/sr and still more preferably 1,700-1,900 W/sr, such as 1,800 W/sr in continuous mode (with the outer/inner radiant intensities *Io,Ii* that are increased by a factor of 2.0-3.0, such as 2.5, in pulsed mode).

The difference between the outer radiant intensity *Io* and the inner radiant intensity *Ii* is mainly due to the difference between the outer half-angle αₒ and the inner half-angle αᵢ (with the lower the half-angle the higher the radiant intensity). Therefore, this result may be achieved even when all the LEDs 415a-415d are of the same type and are supplied with equal or slightly different current intensities; for example, in this case it is possible to obtain an outer radiant intensity *Io* of 0.55-0.57 W/sr and an inner radiant intensity *Ii* of 0.59-0.61 W/sr. Alternatively, the LEDs 310a,310d and the LEDs 310b,310c are of different type and/or the control unit of the imaging apparatus (not shown in the figure) is configured to supply the LEDs 415a-41d with different current intensities (higher for the LEDs 415a,415d and lower for the LEDs 415b,415c). This further increases homogeneity of the field of view (such as compensating for systematic or device-specific alterations).

As a further improvement, the control unit is configured to correct the fluorescence images to reduce any spurious effects of a residual non-homogeneity of the illumination of the field of view. For this purpose, a correction (fluorescence) image is pre-defined. For example, one or more reference images are provided by acquiring corresponding infrared images with an (NIR) camera being sensitive to the wavelength of the excitation light. Each infrared image represents the illumination of the field of view by the illumination unit. Particularly, each basic picture element (pixel) of the infrared image has a value depending on the irradiance of a corresponding location of the field of view. Alternatively, the reference images are provided by acquiring corresponding fluorescence images of a reference object having homogeneous fluorescence, such a calibration phantom, with the imaging apparatus (whose illumination unit provides the excitation light to the reference object). The correction image is generated by setting each pixel value thereof according to an average of the corresponding pixel values of the reference (infrared or fluorescence) images, with the higher the irradiance of the location the lower the pixel value of the correction image (for example, inversely proportional thereto). For each fluorescence image that is acquired, the control unit then multiplies the fluorescence image by the correction image (pixel-by-pixel) before its displaying. In this way, the pixel values of the fluorescence image with lower illumination increase and/or the pixel values of the fluorescence image with higher illumination decrease so as to compensate (at least in part) the residual non-homogeneity of the illumination of the field of view.

With reference now to FIG.5-FIG.7, different examples are shown of experimental results relating to the solution according to an embodiment of the present disclosure.

Each figure comprises an infrared image generated by the illumination unit, with the darker each pixel the higher its irradiance. Moreover, each figure comprises an (irradiance) diagram plotting the irradiance of the field of view of the illumination unit against a distance from its center, in [W/cm²] on the ordinate axis and in [mm] on the abscissa axis, respectively.

Starting from FIG.5, it shows an infrared image 505 and an irradiance diagram 510 obtained with an illumination unit having the above-described structure of FIG.3, but with all the excitation sources equal to each other, particularly, having a same half-angle of 9°. In this case, the irradiance at ±70 mm from the center of the field of view decreases to ~54.5% of its maximum value. Moreover, a distribution of the irradiance exhibits a donut effect with a remarkable depression at the center of the field of view, wherein the irradiance decreases by ~5% of its maximum value (caused by the impossibility of distributing the LEDs uniformly in the illumination unit because of the need of arranging the collection optics of its acquisition unit at the center thereof). Therefore, this structure is not advantageous and acceptable since the illumination is less preforming exactly at the center of the field of view, *i.e.,* where maximum proper illumination is indeed expected to be provided.

Moving to FIG.6, it shows an infrared image 605 and an irradiance diagram 610 obtained with an illumination unit having the same structure of FIG.3 but wherein the 3^{rd} excitation source from the center of the illumination unit in each excitation source alignment now has a different half-angle of 24°. This provides a better homogeneity of the illumination with respect to that of FIG.5, since the irradiance at ±70 mm from the center of the field of view now decreases only to ~57.5% of its maximum value. Moreover, the donut effect in the distribution of the irradiance is substantially removed, with a profile of the irradiance around the center of the field of view that is substantially flat at its maximum value of ~19 mW/cm².

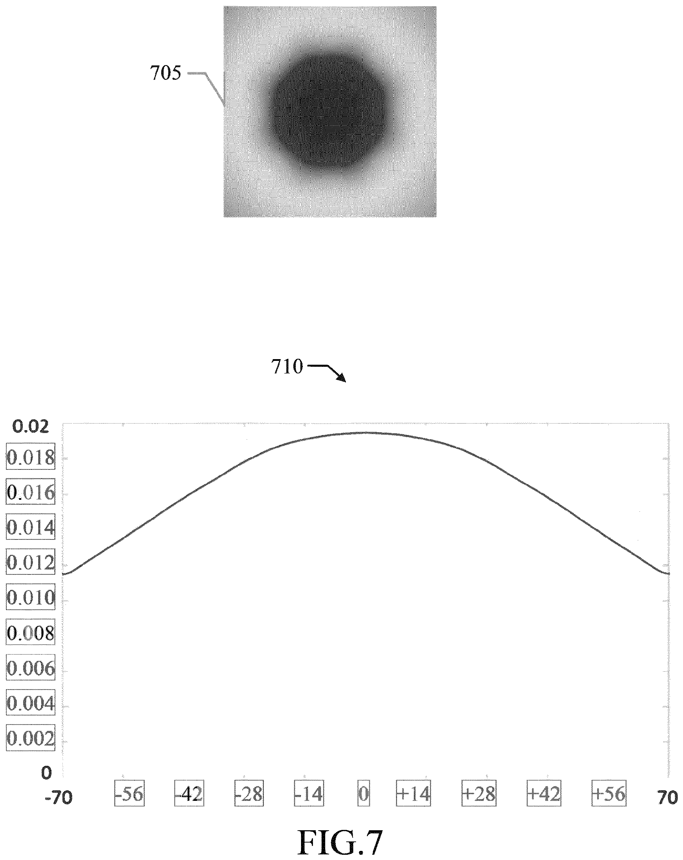

Moving to FIG.7, it shows an infrared image 705 and an irradiance diagram 710 obtained with an illumination unit having the same structure of FIG.3 but wherein the inner excitation sources (2^{nd} and 3^{rd} from the center of the illumination unit) in each excitation source alignment now have a different half-angle of 24°. This provides a better homogeneity of the illumination with respect to that of FIG.6, since the irradiance at ±70 mm from the center of the field of view now decreases only to ~58.9% of its maximum value. Moreover, in this case as well the donut effect in the distribution of the irradiance is substantially removed, with a slightly higher maximum value thereof (~19.5 mW/cm²) at the center of the field of view, but with the profile of the irradiance around the center of the field of view that is less flat than it is in FIG.6.

Therefore, the structure of FIG.6 provides the best distribution of the illumination *(i.e.,* low reduction moving away from the center and flat profile at the center), but it requires an asymmetric arrangement. The structure of FIG.7 instead has a symmetric arrangement providing a good distribution of the illumination *(i.e.,* lower reduction moving away from the center), but with a profile at the center that is less flat.

### Modifications

In order to satisfy local and specific requirements, a person skilled in the art may apply many logical and/or physical modifications and alterations to the present disclosure. More specifically, although this disclosure has been described with a certain degree of particularity with reference to one or more embodiments thereof, it should be understood that various omissions, substitutions and changes in the form and details as well as other embodiments are possible. Particularly, different embodiments of the present disclosure may be practiced even without the specific details (such as the numerical values) set forth in the preceding description to provide a more thorough understanding thereof; conversely, well-known features may have been omitted or simplified in order not to obscure the description with unnecessary particulars. Moreover, it is expressly intended that specific elements and/or method steps described in connection with any embodiment of the present disclosure may be incorporated in any other embodiment as a matter of general design choice. Moreover, items presented in a same group and different embodiments, examples or alternatives are not to be construed as de facto equivalent to each other (but they are separate and autonomous entities). In any case, each numerical value should be read as modified according to applicable tolerances; particularly, unless otherwise indicated, the terms "substantially", "about", "approximately" and the like should be understood as within 10%, preferably 5% and still more preferably 1%. Moreover, each range of numerical values should be intended as expressly specifying any possible number along the *continuum* within the range (comprising its end points). Ordinal or other qualifiers are merely used as labels to distinguish elements with the same name but do not by themselves connote any priority, precedence or order. The terms include, comprise, have, contain, involve and the like should be intended with an open, non-exhaustive meaning *(i.e.,* not limited to the recited items), the terms based on, dependent on, according to, function of and the like should be intended as a non-exclusive relationship *(i.e.,* with possible further variables involved), the term a/an should be intended as one or more items (unless expressly indicated otherwise), and the term means for (or any means-plus-function formulation) should be intended as any structure adapted or configured for carrying out the relevant function.

For example, the invention provides an illumination unit for use in a fluorescence imaging apparatus. However, the fluorescence imaging apparatus may be of any type (see below).

In the invention, the illumination unit has a hole. However, the hole may be of any type (for example, with any shape and size, arranged at any position, either centrally or eccentrically in the illumination unit, and so on).

In the invention, the hole is for receiving collection optics of an acquisition unit of the fluorescence imaging apparatus. However, the collection optics may be of any type (see below) and it may be received in the hole in any way (for example, with interference/clearance fitting, flush/protruding/recessed, with/without any other components and so on).

In the invention, the illumination unit comprises a plurality of excitation sources. However, the excitation sources may be in any number and of any type (for example, based on LEDs, LECs and so on).

In the invention, the excitation sources are for providing an excitation light of one or more fluorescence substances. However, the excitation light may be of any type (for example, NIR, Infra-Red (IR), visible and so on) for exciting any number and type of fluorescence substances (for example, any extrinsic/intrinsic or exogenous/endogenous fluorescence substance, such as a fluorescence agent, a natural fluorescence component and so on).

In the invention, the excitation light has a common wavelength. However, the common wavelength may have any value (with the possibility that is not excluded of having additional excitation sources providing the excitation light with one or more different wavelengths).

In the invention, the excitation sources are arranged in a plurality of alignments. However, the alignments may be in any number and of any length.

In the invention, the alignments extend radially from the hole in a uniform way. However, the alignments may extend in any way (for example, starting at any distance from the hole either the same or different, down to zero, spaced apart by any angle according to their number and so on).

In the invention, each of the alignments comprises a plurality of the excitation sources. However, each alignment may comprise any number of excitation sources (for example, mounted on a common support or on corresponding separate supports, integrated on a common substrate or on corresponding separate substrates, uniformly or non-uniformly spaced along the alignment, and so on).

In the invention, the excitation sources of each alignment are configured to have at least in part different emission characteristics of the excitation light. However, the emission characteristics may be in any number and of any type (for example, emission cones, radiant intensities, orientations with respect to a support surface and so on) and they may differ along each alignment in any way (for example, between inner and outer excitation sources, among two or more alternated groups each of any number of excitation sources and so on).

Further embodiments provide additional advantageous features, which may however be omitted at all in a basic implementation.

In an embodiment, the alignments have a common number of excitation sources. However, the common number may have any value.

In an embodiment, the alignments have a common arrangement of the excitation sources. However, the common arrangement may be of any type (for example, position, spacing and so on).

In an embodiment, the alignments have a common configuration of excitation sources. However, the common configuration may be of any type (for example, emission cones, radiant intensities, orientations and so on).

In any case, the possibility is not excluded of having the alignments that are different at least in part (for example, by maintaining rotational symmetry but with some alignments distributed uniformly that have different numbers of excitation sources, such as one or more outer excitation sources being missing, different distances from the hole, different spacing among the excitation sources and so on).

In an embodiment, in each of the alignments the excitation sources are configured to emit the excitation light with at least in part different emission cones. However, the emission cones may be of any type (for example, symmetric/asymmetric, with any radiation angle and so on).

In an embodiment, in each of the alignments the excitation sources are arranged on a common support surface. However, the support surface may be of any type (for example, a PCB, a heat dissipation plate and the like for each alignment or group thereof, up to a single one for all of them, and so on).

In an embodiment, the emission cones have corresponding axes of symmetry extending perpendicularly to the common support surface. However, the possibility is not excluded of tilting at least part of the excitation sources with respect to the support surface.

In an embodiment, in each of the alignments the excitation sources comprise one or more inner excitation sources and a plurality of outer excitation sources surrounding the inner excitation sources along the alignment. However, the inner/outer excitation sources may be in any number and arranged in any way (for example, with a same number of outer excitation sources at both ends of the alignment, with a different number of excitation sources at the two ends of the alignment and so on).

In an embodiment, the outer excitation sources are configured to emit the excitation light with an outer radiation angle and the inner excitation sources are configured to emit the excitation light with an inner radiation angle strictly higher than the outer radiation angle. However, the outer/inner radiation angles may be defined in any way (for example, at which the irradiance decreases to any fraction of its maximum value, within which there is a specific fraction of the total radiated power at any point and so on) and they may have any values (either in relative or absolute terms).

In an embodiment, the inner radiation angle is equal to 1.9-3.1 times the outer radiation angle. However, the relationship between the inner radiation angle and the outer radiation angle may be defined in any way (for example, by their ratio, difference and so on).

In an embodiment, the inner radiation angle is 16°-28° and the outer radiation angle is 3°-15°. However, the radiation angles may be defined in any way (for example, degrees, radiant and so on).

In an embodiment, the excitation sources comprise corresponding lenses defining the corresponding radiation angles. However, the lenses may be of any type (for example, material, shape and so on); in any case, the possibility is not excluded of obtaining the same result in a different way (for example, with different lenses for corresponding groups of excitation sources, with excitation sources having different structures and so on).

In an embodiment, in each of the alignments the excitation sources are configured to emit the excitation light with at least in part different radiant intensities. However, the radiant intensities may have any values (either in relative or absolute terms) and they may be obtained in any way (for example, as a consequence of the different emission cones of the excitation sources, by supplying the excitation sources with different currents and/or voltages, by providing excitation sources with different structures and so on).

In an embodiment, the inner excitation sources are configured to emit the excitation light with an inner radiant intensity and the outer excitation sources are configured to emit the excitation light with an outer radiant intensity strictly higher than the inner radiant intensity. However, the inner/outer radiant intensities may have any values (either in relative or absolute terms).

In an embodiment, the outer radiant intensity is 1.4-2.3 times the inner radiant intensity. However, the relationship between the outer radiant intensity and the inner radiant intensity may be defined in any way (for example, by their ratio, difference and so on).

In an embodiment, in each of the alignments the excitation sources comprise a first number of first outer excitation sources, a second number (different from the first number) of second outer excitation sources and one or more inner excitation sources comprised between the first outer excitation sources and the second outer excitation sources along the alignment. However, the first/second number may be of any type (for example, with any values (one or more), with the number of the outer excitation sources proximal to the hole being higher or lower than the number of the outer excitation sources distal from the hole, and so on).

In an embodiment, in each of the alignments the excitation sources comprise a common number of first outer excitation sources and of second outer excitation sources, and one or more inner excitation sources comprised between the first outer excitation sources and the second outer excitation sources along the alignment. However, the common number may have any value (one or more).

In an embodiment, the excitation sources comprise corresponding excitation LEDs. However, the excitation LEDs may be of any type (for example, surface-mounting/thru-hole LEDs, OLEDs and so on).

In an embodiment, the illumination unit comprises a plurality of excitation filters corresponding to the alignments. However, the excitation filters may be of any type (for example, band-pass, low-pass, high-pass and the like, with any bandwidth, with any shape, of any material and so on); in any case, the possibility is not excluded of having two or more excitation filters for each alignment, each excitation filter for two or more alignments, down to a single one for all of them.

In an embodiment, each of the excitation filters covers the excitation sources of the corresponding alignment. However, each excitation filter may cover the corresponding excitation sources in any way (for example, by contacting them or a part thereof, spaced apart from them, with a flat structure arranged above the excitation sources, with a concave structure embracing the excitation sources at least in part and so on).

In an embodiment, the illumination unit comprises a plurality of white sources for providing a white light. However, the white sources may be in any number and of any type (for example, either the same or different with respect to the excitation sources) for providing any light causing no significant excitation of the fluorescence substances.

In an embodiment, the white sources are interposed among the alignments in a uniform way. However, the white sources may be interposed among the alignments in any way (for example, with any number of white sources between each pair of adjacent alignments, with the white sources between each pair of adjacent alignments that are arranged in any way, such as radially, circumferentially and the like, at any distance from the center of the illumination unit and so on).

An embodiment provides an imaging head for use in a fluorescence imaging apparatus. However the imaging head may be of any type (for example, mounted on any support structure, hand-held and so on) for use in any fluorescence imaging apparatus (see below).

In an embodiment, the imaging head comprises the illumination unit of above and an acquisition unit. However, the acquisition unit may be of any type (for example, based on any collection optics, with or without a reflectance camera and so on).

In an embodiment, the acquisition unit is for acquiring fluorescence images of a field of view being illuminated by the illumination unit. However, the fluorescence images may be of any type (for example, multi-spectral, mono-chrome and so on) and representing any field of view (for example, relating to any body-part to be operated, analyzed, treated and so on).

In an embodiment, the acquisition unit has collection optics acting through the hole of the illumination unit. However, the collections optics may be of any type (for example, based on EMCCD, CMOS, InGaAs, PMT and the like sensors, with any number and type of lenses, wave guides, mirrors and so on) and it may act through the hole in any way (see above).

An embodiment provides a fluorescence imaging apparatus. However, the fluorescence imaging apparatus may be of any type (for example, a guided surgery equipment, a scanner, a specimen imager and so on).

In an embodiment, the fluorescence imaging apparatus comprises the imaging head of above and a control unit for controlling the imaging head. However, the control unit may be of any type (for example, a microcontroller, a personal computer and so on) and arranged at any position (for example, in a central body of the fluorescence imaging apparatus, in the imaging head and so on).

In an embodiment, the fluorescence imaging apparatus comprises an output unit for displaying the fluorescence images. However, the output unit may be of any type (for example, one or more monitors, virtual reality glasses and so on).

Further embodiments provide additional advantageous features, which may however be omitted at all in a basic implementation.

In an embodiment, the control unit is configured to correct the fluorescence images according to a correction image based on a distribution of an illumination of the field of view by the illumination unit. However, the correction image may be defined in any way (for example, based in any way on any number of images representing the illumination of the field of view, fixed, generated periodically, at the beginning of every imaging procedure and so on) and it may be used to correct the fluorescence images in any way (for example, by a product, an addition/subtraction, a convolution operation and so on).

Generally, similar considerations apply if the illumination unit, the imaging head and the fluorescence imaging apparatus each has a different structure or comprises equivalent components, or it has other operative characteristics (provided that it remains within the scope of the claims). In any case, every component thereof may be separated into more elements, or two or more components may be combined together into a single element; moreover, each component may be replicated to support the execution of the corresponding operations in parallel. Moreover, unless specified otherwise, any interaction between different components generally does not need to be continuous, and it may be either direct or indirect through one or more intermediaries.

An embodiment provides a method for imaging a body-part of a patient in a medical application with the fluorescence imaging apparatus of above. However, the method may be used for imaging any body-part (for example, one or more organs, a region thereof or tissues, in any pathological/health condition and so on) and of any patient (for example, a human being, an animal and so on); moreover, the method may be used in any medical application (for example, surgery, diagnostics, therapy and so on). In any case, although the method may facilitate the task of a physician, it only provides intermediate results that may help him/her but with the medical activity *stricto sensu* that is always made by the physician himself/herself.

In an embodiment, the method comprises illuminating (by the illumination unit) the body-part. However, the body-part may be illuminated in any way (for example, providing only the excitation light, the white light as well and so on).

In an embodiment, the method comprises acquiring (by the acquisition unit) one or more fluorescence images of the body-part being illuminated. However, the fluorescence images may be acquired in any way (for example, alone, together with corresponding reflectance images and so on).

In an embodiment, the method comprises displaying (by the output unit) the fluorescence images. However, the fluorescence images may be displayed in any way (for example, alone, combined with the reflectance images, in real-time, off-line and so on).

Further embodiments provide additional advantageous features, which may however be omitted at all in a basic implementation.

In an embodiment, the method comprises correcting (by the control unit) the fluorescence images according to a correction image based on a distribution of an illumination of the field of view by the illumination unit. However, the fluorescence images may be corrected in any way (see above).

Generally, the method may be implemented in any way (with the same considerations as above that apply by analogy to the corresponding steps).

A computer program is proided, which is configured for causing the control unit of the fluorescence imaging apparatus of above to perform the same method when the computer program is executed on the control unit. A computer program product is provided, which comprises a computer readable storage medium embodying a computer program, the computer program being loadable into a working memory of the control unit of the fluorescence imaging apparatus of above thereby configuring the control unit to perform the same method. However, the program may be used on the control unit of the fluorescence imaging apparatus of above, or more generally on any control unit of any fluorescence imaging apparatus (even of standard type). In any case, the same solution may be implemented with a hardware structure (for example, by electronic circuits integrated in one or more chips of semiconductor material), or with a combination of software and hardware suitably programmed or otherwise configured.

## Claims

1. An illumination unit (218), for use in an imaging head (165) of a fluorescence imaging apparatus (100) configured for imaging a scene comprised in a field of view thereof, for illuminating the scene of the field of view, wherein the illumination unit (218) has a hole (218h) for receiving collection optics (224) of an acquisition unit (221) of the fluorescence imaging apparatus (100) for acquiring fluorescence images of the scene of the field of view and comprises a plurality of excitation sources (310) for providing an excitation light of one or more fluorescence substances having a common wavelength, the excitation sources (310) being arranged in a plurality of alignments (315) extending radially from the hole (218h) in a uniform way,
**characterized in that**
each of the alignments (315) comprises a plurality of the excitation sources (310a-310d) being configured to have at least in part different emission characteristics of the excitation light for increasing an illumination homogeneity of the field of view.

2. The illumination unit (218) according to claim 1, wherein the alignments (315) have a common number of the excitation sources (310a-310d) with a common arrangement and a common configuration of the excitation sources (310a-310d).

3. The illumination unit (218) according to claim 1 or 2, wherein in each of the alignments (315) the excitation sources (310) comprise one or more inner excitation sources (310b,310c) and a plurality of outer excitation sources (310a,310d) surrounding the inner excitation sources (310b,310c) along the alignment (315), the outer excitation sources (310a,310d) being configured to emit the excitation light with an outer radiation angle (αₒ) and the inner excitation sources (310b,310c) being configured to emit the excitation light with an inner radiation angle (αᵢ) strictly higher than the outer radiation angle (αₒ).

4. The illumination unit (218) according to claim 3, wherein in each of the alignments (315) the excitation sources (310a-310d) are arranged on a common support surface (410s), the excitation sources (310) being configured to emit the excitation light with corresponding at least in part different emission cones (405a-405d) having corresponding axes of symmetry (Xa-Xd) extending perpendicularly to the common support surface (410s).

5. The illumination unit (218) according to claim 3 or 4, wherein the inner radiation angle (αᵢ) is 16°-28° and the outer radiation angle is 3°-15° (αₒ).

6. The illumination unit (218) according to any claim from 3 to 5, wherein the excitation sources (310a-310d) comprise corresponding excitation LEDs (415a-415d) and corresponding lenses (420a-420d) defining the corresponding radiation angles (αᵢ,αₒ).

7. The illumination unit (218) according to any claim from 1 to 6, wherein in each of the alignments (315) the excitation sources (310) comprise one or more inner excitation sources (310b,310c) and a plurality of outer excitation sources (310a,310d) surrounding the inner excitation sources (310b,310c) along the alignment (315), the inner excitation sources (310b,310c) being configured to emit the excitation light with an inner radiant intensity and the outer excitation sources (310a,310d) being configured to emit the excitation light with an outer radiant intensity strictly higher than the inner radiant intensity.

8. The illumination unit (218) according to any claim from 1 to 7, wherein in each of the alignments (315) the excitation sources (310d) comprise a first number of first outer excitation sources (310a), a second number different from the first number of second outer excitation sources (310d) and one or more inner excitation sources (310b,310c) comprised between the first outer excitation sources (310a) and the second outer excitation sources (310d) along the alignment (315).

9. The illumination unit (218) according to any claim from 1 to 7, wherein in each of the alignments (315) the excitation sources (310d) comprise a common number of first outer excitation sources (310a) and of second outer excitation sources (310d), and one or more inner excitation sources (310b,310c) comprised between the first outer excitation sources (310a) and the second outer excitation sources (310d) along the alignment (315).

10. The illumination unit (218) according to any claim from 1 to 9, wherein the illumination unit (218) comprises a plurality of excitation filters (320) corresponding to the alignments (315), each of the excitation filters (320) covering the excitation sources (310a-310d) of the corresponding alignment (315).

11. The illumination unit (218) according to any claim from 1 to 10, wherein the illumination unit (218) comprises a plurality of white sources (325) for providing a white light, the white sources (325) being interposed among the alignments (315) in a uniform way.

12. An imaging head (165) for use in a fluorescence imaging apparatus (100), the imaging head comprising the illumination unit (218) according to any claim from 1 to 11 and an acquisition unit (221) for acquiring fluorescence images of the scene of the field of view (203), the acquisition unit (221) having collection optics (224) acting through the hole (218h) of the illumination unit (218).

13. A fluorescence imaging apparatus (100) comprising the imaging head (165) according to claim 12, a control unit (115) for controlling the imaging head (165) and an output unit (140,155) for displaying the fluorescence images.

14. The fluorescence imaging apparatus (100) according to claim 13, wherein the control unit (115) is configured to correct the fluorescence images according to a correction image based on a distribution of an illumination of the field of view (203) by the illumination unit (218).

15. A method for imaging a body-part (209) of a patient (206) in a medical application with the fluorescence imaging apparatus (100) of claim 13, wherein the method comprises:
illuminating, by the illumination unit (218), the body-part (209),
acquiring, by the acquisition unit (221), one or more fluorescence images of the body-part (209) being illuminated, and
displaying, by the output unit (140,155), the fluorescence images.

## Patentansprüche

1. Beleuchtungseinheit (218) zur Verwendung in einem Bildgebungskopf (165) einer Fluoreszenzbildgebungsvorrichtung (100), die zum Bildgeben einer Szene konfiguriert ist, die in einem Sichtfeld davon umfasst ist, zum Beleuchten der Szene des Sichtfelds, wobei die Beleuchtungseinheit (218) ein Loch (218h) zum Aufnehmen einer Sammeloptik (224) einer Erfassungseinheit (221) der Fluoreszenzbildgebungsvorrichtung (100) zum Erfassen von Fluoreszenzbildern der Szene des Sichtfelds aufweist und eine Vielzahl von Anregungsquellen (310) zum Bereitstellen eines Anregungslichts einer oder mehrerer Fluoreszenzsubstanzen mit einer gemeinsamen Wellenlänge umfasst, wobei die Anregungsquellen (310) in einer Vielzahl von Ausrichtungen (315) angeordnet sind, die sich gleichmäßig radial von dem Loch (218h) erstrecken,
**dadurch gekennzeichnet, dass**
jede der Ausrichtungen (315) eine Vielzahl von Anregungsquellen (310a-310d) umfasst, die dazu konfiguriert sind, mindestens teilweise unterschiedliche Emissionseigenschaften des Anregungslichts aufzuweisen, um eine Beleuchtungshomogenität des Sichtfelds zu erhöhen.

2. Beleuchtungseinheit (218) nach Anspruch 1, wobei die Ausrichtungen (315) eine gemeinsame Anzahl der Anregungsquellen (310a-310d) mit einer gemeinsamen Anordnung und einer gemeinsamen Konfiguration der Anregungsquellen (310a-310d) aufweisen.

3. Beleuchtungseinheit (218) nach Anspruch 1 oder 2, wobei in jeder der Ausrichtungen (315) die Anregungsquellen (310) eine oder mehrere innere Anregungsquellen (310b,310c) und eine Vielzahl von äußeren Anregungsquellen (310a,310d) umfassen, die die inneren Anregungsquellen (310b,310c) entlang der Ausrichtung (315) umgeben, wobei die äußeren Anregungsquellen (310a,310d) dazu konfiguriert sind, das Anregungslicht mit einem äußeren Strahlungswinkel (αₒ) zu emittieren, und die inneren Anregungsquellen (310b,310c) dazu konfiguriert sind, das Anregungslicht mit einem inneren Strahlungswinkel (αᵢ) zu emittieren, der deutlich höher als der äußere Strahlungswinkel (αₒ) ist.

4. Beleuchtungseinheit (218) nach Anspruch 3, wobei in jeder der Ausrichtungen (315) die Anregungsquellen (310a-310d) auf einer gemeinsamen Trägeroberfläche (410s) angeordnet sind, wobei die Anregungsquellen (310) dazu konfiguriert sind, das Anregungslicht mit entsprechenden, mindestens teilweise unterschiedlichen Emissionskegeln (405a-405d) mit entsprechenden Symmetrieachsen (Xa-Xd), die sich senkrecht zu der gemeinsamen Trägeroberfläche (410s) erstrecken, zu emittieren.

5. Beleuchtungseinheit (218) nach Anspruch 3 oder 4, wobei der innere Strahlungswinkel (αᵢ) 16°-28° beträgt und der äußere Strahlungswinkel 3°-15° (αₒ) beträgt.

6. Beleuchtungseinheit (218) nach einem der Ansprüche 3 bis 5, wobei die Anregungsquellen (310a-310d) entsprechende Anregungs-LEDs (415a-415d) und entsprechende Linsen (420a-420d), die die entsprechenden Strahlungswinkel (αᵢ,αₒ) definieren, umfassen.

7. Beleuchtungseinheit (218) nach einem der Ansprüche 1 bis 6, wobei in jeder der Ausrichtungen (315) die Anregungsquellen (310) eine oder mehrere innere Anregungsquellen (310b,310c) und eine Vielzahl von äußeren Anregungsquellen (310a,310d), die die inneren Anregungsquellen (310b,310c) entlang der Ausrichtung (315) umgeben, umfassen,wobei die inneren Anregungsquellen (310b,310c) dazu konfiguriert sind, das Anregungslicht mit einer inneren Strahlungsintensität zu emittieren, und die äußeren Anregungsquellen (310a,310d) dazu konfiguriert sind, das Anregungslicht mit einer äußeren Strahlungsintensität zu emittieren, die deutlich höher als die innere Strahlungsintensität ist.

8. Beleuchtungseinheit (218) nach einem der Ansprüche 1 bis 7, wobei in jeder der Ausrichtungen (315) die Anregungsquellen (310d) eine erste Anzahl von ersten äußeren Anregungsquellen (310a), eine zweite Anzahl, die sich von der ersten Anzahl unterscheidet, von zweiten äußeren Anregungsquellen (310d) und eine oder mehrere innere Anregungsquellen (310b,310c), die zwischen den ersten äußeren Anregungsquellen (310a) und den zweiten äußeren Anregungsquellen (310d) entlang der Ausrichtung (315) umfasst sind, umfassen.

9. Beleuchtungseinheit (218) nach einem der Ansprüche 1 bis 7, wobei in jeder der Ausrichtungen (315) die Anregungsquellen (310d) eine gemeinsame Anzahl von ersten äußeren Anregungsquellen (310a) und von zweiten äußeren Anregungsquellen (310d) und eine oder mehrere innere Anregungsquellen (310b,310c), die zwischen den ersten äußeren Anregungsquellen (310a) und den zweiten äußeren Anregungsquellen (310d) entlang der Ausrichtung (315) umfasst sind, umfassen.

10. Beleuchtungseinheit (218) nach einem der Ansprüche 1 bis 9, wobei die Beleuchtungseinheit (218) eine Vielzahl von Anregungsfiltern (320) umfasst, die den Ausrichtungen (315) entsprechen, wobei jeder der Anregungsfilter (320) die Anregungsquellen (310a-310d) der entsprechenden Ausrichtung (315) bedeckt.

11. Beleuchtungseinheit (218) nach einem der Ansprüche 1 bis 10, wobei die Beleuchtungseinheit (218) eine Vielzahl von weißen Quellen (325) zum Bereitstellen eines weißen Lichts umfasst, wobei die weißen Quellen (325) gleichmäßig zwischen den Ausrichtungen (315) zwischengeschaltet sind.

12. Bildgebungskopf (165) zur Verwendung in einer Fluoreszenzbildgebungsvorrichtung (100), wobei der Bildgebungskopf die Beleuchtungseinheit (218) nach einem der Ansprüche 1 bis 11 und eine Erfassungseinheit (221) zum Erfassen von Fluoreszenzbildern der Szene des Sichtfelds (203) umfasst, wobei die Erfassungseinheit (221) eine Sammeloptik (224) aufweist, die durch das Loch (218h) der Beleuchtungseinheit (218) wirkt.

13. Fluoreszenzbildgebungsvorrichtung (100), umfassend den Bildgebungskopf (165) nach Anspruch 12, eine Steuereinheit (115) zum Steuern des Bildgebungskopfs (165) und eine Ausgabeeinheit (140,155) zum Anzeigen der Fluoreszenzbilder.

14. Fluoreszenzbildgebungsvorrichtung (100) nach Anspruch 13, wobei die Steuereinheit (115) dazu konfiguriert ist, die Fluoreszenzbilder gemäß einem Korrekturbild basierend auf einer Verteilung einer Beleuchtung des Sichtfelds (203) durch die Beleuchtungseinheit (218) zu korrigieren.

15. Verfahren zur Bildgebung eines Körperteils (209) eines Patienten (206) in einer medizinischen Anwendung mit der Fluoreszenzbildgebungsvorrichtung (100) nach Anspruch 13, wobei das Verfahren Folgendes umfasst:
Beleuchten des Körperteils (209) durch die Beleuchtungseinheit (218),
Erfassen, durch die Erfassungseinheit (221), eines oder mehrerer Fluoreszenzbilder des Körperteils (209), das beleuchtet wird, und
Anzeigen der Fluoreszenzbilder durch die Ausgabeeinheit (140,155).

## Revendications

1. Unité d'éclairage (218), destinée à être utilisée dans une tête d'imagerie (165) d'un appareil d'imagerie par fluorescence (100) configuré pour former l'image d'une scène comprise dans un champ de vision de celle-ci, pour éclairer la scène du champ de vision, ladite unité d'éclairage (218) comportant un trou (218h) pour recevoir une optique de collecte (224) d'une unité d'acquisition (221) de l'appareil d'imagerie par fluorescence (100) pour acquérir des images de fluorescence de la scène du champ de vision et comprenant une pluralité de sources d'excitation (310) destinées à fournir une lumière d'excitation d'une ou plusieurs substances de fluorescence possédant une longueur d'onde commune, les sources d'excitation (310) étant agencées dans une pluralité d'alignements (315) s'étendant radialement à partir du trou (218h) d'une manière uniforme,
**caractérisée en ce que**
chacun des alignements (315) comprend une pluralité de sources d'excitation (310a-310d) configurées pour posséder au moins en partie des caractéristiques d'émission différentes de la lumière d'excitation pour augmenter une homogénéité d'éclairage du champ de vision.

2. Unité d'éclairage (218) selon la revendication 1, lesdits alignements (315) possédant un nombre commun de sources d'excitation (310a-310d) avec un agencement commun et une configuration commune des sources d'excitation (310a-310d).

3. Unité d'éclairage (218) selon la revendication 1 ou 2, dans chacun des alignements (315), lesdites sources d'excitation (310) comprenant une ou plusieurs sources d'excitation internes (310b,310c) et une pluralité de sources d'excitation externes (310a,310d) entourant les sources d'excitation internes (310b,310c) le long de l'alignement (315), les sources d'excitation externes (310a,310d) étant configurées pour émettre la lumière d'excitation avec un angle de rayonnement externe (αₒ) et les sources d'excitation internes (310b,310c) étant configurées pour émettre la lumière d'excitation avec un angle de rayonnement interne (αᵢ) strictement supérieur à l'angle de rayonnement externe (αₒ).

4. Unité d'éclairage (218) selon la revendication 3, dans chacun des alignements (315), lesdites sources d'excitation (310a-310d) étant agencées sur une surface de support commune (410s), les sources d'excitation (310) étant configurées pour émettre la lumière d'excitation avec des cônes d'émission correspondants au moins en partie différents (405a-405d) possédant des axes de symétrie correspondants (Xa-Xd) s'étendant perpendiculairement à la surface de support commune (410s).

5. Unité d'éclairage (218) selon la revendication 3 ou 4, ledit angle de rayonnement interne (αᵢ) étant 16°-28° et ledit angle de rayonnement externe étant 3°-15° (αₒ).

6. Unité d'éclairage (218) selon l'une quelconque des revendications 3 à 5, lesdites sources d'excitation (310a-310d) comprenant des LED d'excitation correspondantes (415a-415d) et des lentilles correspondantes (420a-420d) définissant les angles de rayonnement correspondants (αᵢ,αₒ).

7. Unité d'éclairage (218) selon l'une quelconque des revendications 1 à 6, dans chacun des alignements (315), lesdites sources d'excitation (310) comprenant une ou plusieurs sources d'excitation internes (310b,310c) et une pluralité de sources d'excitation externes (310a,310d) entourant les sources d'excitation internes (310b,310c) le long de l'alignement (315), les sources d'excitation internes (310b,310c) étant configurées pour émettre la lumière d'excitation avec une intensité de rayonnement interne et les sources d'excitation externes (310a,310d) étant configurées pour émettre la lumière d'excitation avec une intensité de rayonnement externe strictement supérieure à l'intensité de rayonnement interne.

8. Unité d'éclairage (218) selon l'une quelconque des revendications 1 à 7, dans chacun des alignements (315), lesdites sources d'excitation (310d) comprenant un premier nombre de premières sources d'excitation externes (310a), un second nombre différent du premier nombre de secondes sources d'excitation externes (310d) et une ou plusieurs sources d'excitation internes (310b,310c) comprises entre les premières sources d'excitation externes (310a) et les secondes sources d'excitation externes (310d) le long de l'alignement (315).

9. Unité d'éclairage (218) selon l'une quelconque des revendications 1 à 7, dans chacun des alignements (315), lesdites sources d'excitation (310d) comprenant un nombre commun de premières sources d'excitation externes (310a) et de secondes sources d'excitation externes (310d), et une ou plusieurs sources d'excitation internes (310b,310c) comprises entre les premières sources d'excitation externes (310a) et les secondes sources d'excitation externes (310d) le long de l'alignement (315).

10. Unité d'éclairage (218) selon l'une quelconque des revendications 1 à 9, ladite unité d'éclairage (218) comprenant une pluralité de filtres d'excitation (320) correspondant aux alignements (315), chacun des filtres d'excitation (320) recouvrant les sources d'excitation (310a-310d) de l'alignement correspondant (315).

11. Unité d'éclairage (218) selon l'une quelconque des revendications 1 à 10, ladite unité d'éclairage (218) comprenant une pluralité de sources blanches (325) destinées à fournir une lumière blanche, les sources blanches (325) étant interposées parmi les alignements (315) d'une manière uniforme.

12. Tête d'imagerie (165) destinée à être utilisée dans un appareil d'imagerie par fluorescence (100), la tête d'imagerie comprenant l'unité d'éclairage (218) selon l'une quelconque des revendications 1 à 11 et une unité d'acquisition (221) destinée à acquérir des images de fluorescence de la scène du champ de vision (203), l'unité d'acquisition (221) comportant une optique de collecte (224) agissant à travers le trou (218h) de l'unité d'éclairage (218).

13. Appareil d'imagerie par fluorescence (100) comprenant la tête d'imagerie (165) selon la revendication 12, une unité de commande (115) destinée à commander la tête d'imagerie (165) et une unité de sortie (140,155) destinée à afficher les images de fluorescence.

14. Appareil d'imagerie par fluorescence (100) selon la revendication 13, ladite unité de commande (115) étant configurée pour corriger les images de fluorescence selon une image de correction sur la base d'une distribution d'un éclairage du champ de vision (203) par l'unité d'éclairage (218).

15. Procédé permettant l'imagerie d'une partie de corps (209) d'un patient (206) dans une application médicale avec l'appareil d'imagerie par fluorescence (100) de la revendication 13, ledit procédé comprenant :
l'éclairage, par l'unité d'éclairage (218), de la partie de corps (209),
l'acquisition, par l'unité d'acquisition (221), d'une ou plusieurs images de fluorescence de la partie de corps (209) qui est éclairée, et
l'affichage, par l'unité de sortie (140,155), des images de fluorescence.
